Europäisches Patentamt

European Patent Office

Office européen des brevets

(11) Veröffentlichungsnummer: **0 585 652 A2**

## EUROPÄISCHE PATENTANMELDUNG

(21) Anmeldenummer: 93112650.2

(22) Anmeldetag: 06.08.93

(51) Int. Cl.5: **C12N 15/78**, C12P 3/00,
C12S 13/00, C12N 15/52,
C12N 1/21, //(C12N1/21,
C12R1:40)

(30) Priorität: 23.08.92 DE 4227769

(43) Veröffentlichungstag der Anmeldung:
09.03.94 Patentblatt 94/10

(84) Benannte Vertragsstaaten:
BE DE DK ES FR GB IT NL

(71) Anmelder: Gesellschaft für
Biotechnologische Forschung mbH (GBF)
Mascheroder Weg 1
D-38124 Braunschweig(DE)

(72) Erfinder: Horn, Joanne M.
Univ. of West Florida
Pensacola, FL 32514(US)
Erfinder: Brunke, Maren
Mascheroder Weg 1
D-38124 Braunschweig(DE)
Erfinder: Deckwer, Wolf-Dieter
Mascheroder Weg 1
D-38124 Braunschweig(DE)
Erfinder: Timmis, Kenneth N.
Mascheroder Weg 1
D-38124 Braunschweig(DE)

(74) Vertreter: Boeters, Hans Dietrich, Dr. et al
Patentanwälte Boeters Bauer Koepe
Bereiteranger 15
D-81541 München (DE)

(54) Bakterienstämme für die Ouecksilber-Reduktion sowie Verfahren zu ihrer Herstellung.

(57) Die Erfindung betrifft Bakterienstämme für die Quecksilber-Reduktion sowie ein Verfahren zu ihrer Herstellung.

EP 0 585 652 A2

EP 0 585 652 A2

Die biologische Grundlage der Quecksilber- (Hg-) Toxizität ist die Fähigkeit von Hg in seiner zweiwertigen kationischen Form [Hg(II)], Sulfhydryl-, Thioether- und Imidazolgruppen zu binden und dadurch Enzyme zu inaktivieren (26). Es ist deshalb allgemein anerkannt, daß das Hg(II) die am stärksten toxische ionische Form des Metalls ist. Organische Quecksilber-Spezies, und zwar sowohl Alkyl- als auch Arylderivate, können in den Geweben höherer Organismen akkumulieren, wo sie systemische Krankheiten verursachen (17). Trotz der extremen Biotoxizität von Hg(II) und seinen organischen Derivaten werden jedoch schätzungsweise jährlich 11.000 Tonnen durch Menschenhand mobilisiertes Hg und Hg-haltige Verbindungen in die Biosphäre entlassen. Es läßt sich also sagen, daß Quecksilberverschmutzungen ein ernstzunehmendes Risiko für die Umwelt darstellen.

Da die Gesamtmenge an vorhandenem Hg endgültig und unveränderbar ist, kann Abhilfe bezüglich der Hg-Belastungen nur dadurch erzielt werden, daß die ionische Form in eine weniger toxische Spezies überführt wird und/oder daß sie abgetrennt wird, idealerweise in einer für weitere Verwendung in den Kreislauf rückführbaren Gestalt. Derzeit übliche Hg-Beseitigungs-Technologien setzen Adsorptionsmatrices ein, um Quecksilberverbindungen aus industriellen Abgasen oder Abwässern zu entfernen. Durch dieses Verfahren wird jedoch ein fester Sekundärabfall erzeugt, und gebundenes Hg ist für eine weitere Verwendung nicht mehr verfügbar. Das Verfahren weist weiterhin den Nachteil auf, daß es relativ unselektiv ist; auch andere Abfallbestandteile als Hg werden adsorbiert.

Eine alternative Strategie zur Beseitigung, die derzeit untersucht wird, schließt die Umsetzung von Hg(II) in die inertere, flüchtige Elementarform [Hg(0)] ein, und zwar unter Einsatz des Mechanismus der bakteriellen Hg-Resistenz, die auf der Reduktion von Hg(II) zu Hg(0) durch die Aktivität einer cytosolischen Quecksilber-Reduktase beruht (8, 36). Solche bakteriellen Reduktionssysteme könnten entweder an Ort und Stelle für die Beseitigung eingesetzt werden, da flüchtig gemachtes Hg(0) sowohl weniger toxisch als auch weniger bioverfügbar ist, oder aber innerhalb von Bioreaktoren, aus denen das reduzierte Hg(0) bei Bedarf wiedergewonnen werden könnte.

An der spezifischen bakteriellen Quecksilberresistenz (Hg$^r$) sind zum einen die Quecksilber-Reduktase und zum anderen, da Hg(II) auch Membranproteine inaktiviert, ein Hg-spezifisches Transportsystem beteiligt, das die Membran und andere zelluläre Komponenten durch die Beförderung des Hg von der Zelloberfläche zur intrazellulären Quecksilber-Reduktase schützt (24).

Hg$^r$ mit engerem Wirkungsspektrum verleiht Resistenz nur gegenüber Quecksilber-Ionen. An der Quecksilberresistenz mit breitem Wirkungspektrum ist neben den Transport- und Reduktase-Enzymen eine Organoquecksilber-Lyase beteiligt, die Kohlenstoff-Hg-Bindungen protonolytisch spaltet (2, 36, 41). Die Hg$^r$-Systeme mit breitem Wirkungsspektrum verleihen deshalb durch die aufeinanderfolgenden Aktivitäten von Lyase und Reduktase auch Resistenz gegenüber Organoquecksiber-Verbindungen.

Man hat die Hg$^r$-Mechanismen für sowohl engeres als auch breites Wirkungsspektrum in weitem Umfang in Bakterien gefunden. Die Hg$^r$-Strukturgene sind in einem einzigen Operon codiert, das primär durch das merR-Genprodukt reguliert wird, welches seinerseits angrenzend an das merTPABD-Operon transkribiert wird, jedoch in umgekehrter Richtung davon. Die merT- und merP-Genprodukte sind an der Aufnahme und am Transport von Hg(II) beteiligt, merA spezifiziert die Quecksilber-Reduktase, und merB codiert für die Organoquecksilber-Lyase (39 enthält eine Übersicht). merD, das am weitesten vom Promotor entfernt gelegene Gen, das identifiziert wurde, wurde mit einer Koregulations-Funktion bei der Transkription in Verbindung gebracht (22, 29).

Es ist nun Aufgabe der Erfindung, Bakterienstämme mit verbesserten Quecksilber-Entgiftungseigenschaften zur Verfügung zu stellen.

Diese Aufgabe wurde dadurch gelöst, daß Stämme von Pseudomonas putida zur Verfügung gestellt werden, welche infolge konstitutiver Hyperexpression der merTPAB-Gene derartige verbesserte Quecksilber-Entgiftungs-Eigenschaften aufweisen. Erfindungsgemäß werden auch Organismen bereitgestellt, bei denen die Fähigkeit, Hg unter hohen Konzentrationen in erhöhten Raten zu reduzieren, auf genetischer Ebene mit einem Benzol-Katabolismus kombiniert ist. Die entsprechenden Pseudomonas putida-Derivate sind in der Lage, die chemisch ungleichen Bestandteile einer Organoquecksilber-Verbindung, nämlich Phenylquecksilberacetat (PMA), in ihre metallischen und aromatischen Bestandteile zu zerlegen und jeweils getrennt zu entgiften. Die Charakterisierung dieser Isolate zeigt, daß sie für die Behandlung von Hg-haltigen Verunreinigungen geeignet sind.

Die bereitgestellten Stämme sind dadurch gekennzeichnet, daß sie

(a) in der Lage sind, Quecksilber(II)-Ionen aus Quecksilbersalzen und Organoquecksilberverbindungen zu reduzieren,

(b) hohe Resistenz gegenüber Quecksilberverbindungen aufweisen,

(c) einen Expressionsapparat für die quecksilberentgiftenden Proteine besitzen, der völlig unabhängig von der umgebenden Quecksilberkonzentration ist,

2

(d) genetisch stabile Insertionen von überexprimierenden Genen für die Quecksilberresistenz in das Wirtschromosom aufweisen, und

(e) fakultativ zusätzlich Toluol- und Benzolgruppen abbauen können.

Diese Stämme entgiften organische Quecksilberverbindungen wie auch Quecksilbersalze nicht nur bei sehr hohen, sondern auch bei sehr niedrigen Quecksilber-Konzentrationen, welch letztere normalerweise, z.B. in den Wildtypen, nicht zur Induktion der Synthese der quecksilberentgiftenden Proteine ausreichen. Diejenigen Stämme, die auch Benzol- und Toluolgruppen abbauen können, setzen diese Gruppen im wesentlichen letztendlich zu Kohlendioxid und Wasser um, unabhängig davon, ob das Benzol und/oder Toluol als organische Gruppe in einer Quecksilberverbindung oder als Verbindung an sich in die Umgebung des abbauenden Organismus gelangt ist.

Zur Erzeugung der Mutanten wird Pseudomonas putida vorzugsweise mit einem Mini-Transposon ungezielt mutagenisiert, das merTPAB enthält, also Strukturgene, die die Quecksilberresistenz spezifizieren. Das Transposon sollte bevorzugt eine oder mehrere der folgenden Eigenschaften aufweisen:

(i) Eine sehr kurze Endsequenz (z.B. 19 Bp)

(ii) Nur einmaliges Transponieren in den Akzeptor bzw. Rezipienten; die transponierte DNA sollte stabil in den Wirt integriert sein, nicht wandern, sich nicht verdoppeln und auch nicht durch Aktion eines ihrer eigenen Bestandteile wieder herausgeschnitten werden.

(iii) Das Transposon-Konstrukt sollte keine mer-Regulatorgene aufweisen.

Wird ein derartiges Transposon strangabwärts (in 3 `-Richtung) zu solchen Regionen der Wirts-DNA insertiert, die hohe Transkriptionsraten bewirken, so können die Gene wegen des geringen Abstandes zu den starken Wirtspromotoren durch diese exprimiert werden, da die RNA-Polymerase an den Promotor bindet und durch das kurze Zwischenstück hindurch das mer-Gen abliest. Durch die hohe Transkriptionsrate wird eine hohe Kopienzahl des RNA-Transkripts erzeugt, das für die Proteine codiert, die für die (Organo-)Quecksilber-Resistenz und Entgiftung verantwortlich sind.

Im Anschluß an das Mating der Zellen werden selektiv diejenigen Stämme isoliert, die merTPAB konstitutiv hyperexprimieren, was ihnen die Fähigkeit gibt, Hg in erhöhten Raten unter Bedingungen hoher Quecksilberkonzentrationen aus einer organischen Gruppe abzuspalten und das freigesetzte Hg(II) in die weniger toxische und biologisch nicht verwertbare Form [Hg(0)] zu überführen.

In einer zweiten Ausführungsform werden diese Eigenschaften genetisch mit einem Benzol-/Toluol-Katabolismus gekoppelt, sodaß Mikroorganismen zur Verfügung gestellt werden, die nicht nur die Metallkomponente von Phenylquecksilberacetat (PMA) reduzieren können, sondern auch den vollständigen Abbau von dessen aromatischer Gruppe ermöglichen. Diese Isolate sind deshalb fähig, PMA als einzige Kohlenstoff- und Energiequelle zu nutzen.

Die zweckgerichtete Anpassung metabolischer Abläufe durch "genetic engineering" eröffnet die Möglichkeit, verbesserte Mittel für die Entgiftung von Umweltverschmutzungen bereitzustellen. Man hat verschiedene Strategien eingesetzt, um dieses Ziel zu erreichen. Es ist gelungen, Zwischenprodukte des Katabolismus durch Heranziehen alternativer katabolischer Pathways zu kanalisieren, wie auch "nicht erlaubte" Schritte im Katabolismus durch sequentielle Mutationsselektion zu eliminieren, wodurch die Expansion des Pathway-Substratprofils ermöglicht wird (32, 34, 42). Die vorliegende Erfindung zeigt einen anderen Lösungsweg auf: Die enzymatische Spaltung von chemisch ungleichen Einheiten einer Verbindungsklasse, nämlich eines Organometalls, in die Einzelbestandteile, Hg(II) und Benzol, und die Verbindung von Pathways, um jeden der gebildeten Bestandteile getrennt zu entgiften.

Die Erfindung richtet sich dementsprechend sowohl auf konstitutiv hyperexprimierende Quecksilber-Entgiftungsdeterminanten zur Erzeugung von gegen (Organo-)Quecksilber hochresistente Bakterienstämme, die ihre Zielverbindungen unter erhöhten Hg-Konzentrationen in erhöhten Raten entgiften können, als auch auf die Verknüpfung dieser Eigenschaften mit dem Abbau von Benzol, sodaß Quecksilber-Benzol-Derivate vollständig abgebaut werden können.

Während sich früher Änderungen der Regulation in der Synthese katabolischer Enzyme auf die Modifizierung der Effektor-Eigenschaften bestimmter Regulator-Proteine konzentrierte (32, 42), wird die konstitutive Hyperexpression von Hg$^r$ mitbreitem Wirkungsspektrum erfindungsgemäß durch Eliminierung der regulatorischen Kontrolle für mer und das ungezielte Mutagenisieren von P. putida-Wirten mit einem merTPAB-haltigen Mini-Tn5-Element erreicht. Es wurde vorausgesetzt, daß die Transkription von mer-Genen durch Promotoren des exogenen Wirts ausgelöst und durch abgekürzte IS50-Sequenzen gelesen wird.

Das Plasmid pHG106 enthält ein 4,2 kB langes HindIII-Fragment, das die merTPAB-DNA für die Konstruktion des durch pUTHg codierten mini-Tn5 (11, 12) lieferte. Die strangaufwärts gelegene HindIII-Stelle, die für die Konstruktion von sowohl pHG106 als auch pUTHg verwendet wurde, könnte jedoch eine Deletion der -35- und der primären -10-Sequenzen des merTPABD-Promotors (Pmer) aufweisen (16, 28).

Die für merTPAB codierende DNA in pUTHg weist mit Sicherheit Deletion sowohl des merR- als auch des merD-Regulatorgens auf (11, 12), und so könnten ihr auch Elemente der primären Transkriptionskontrolle fehlen.

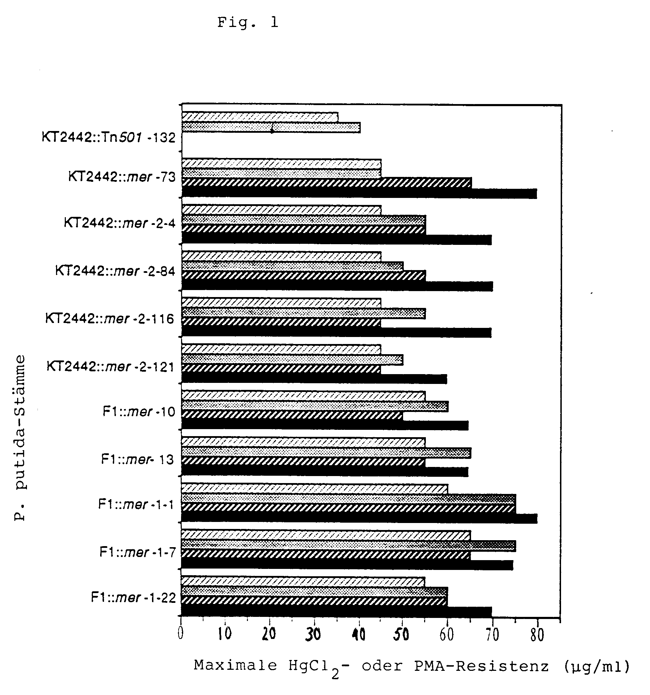

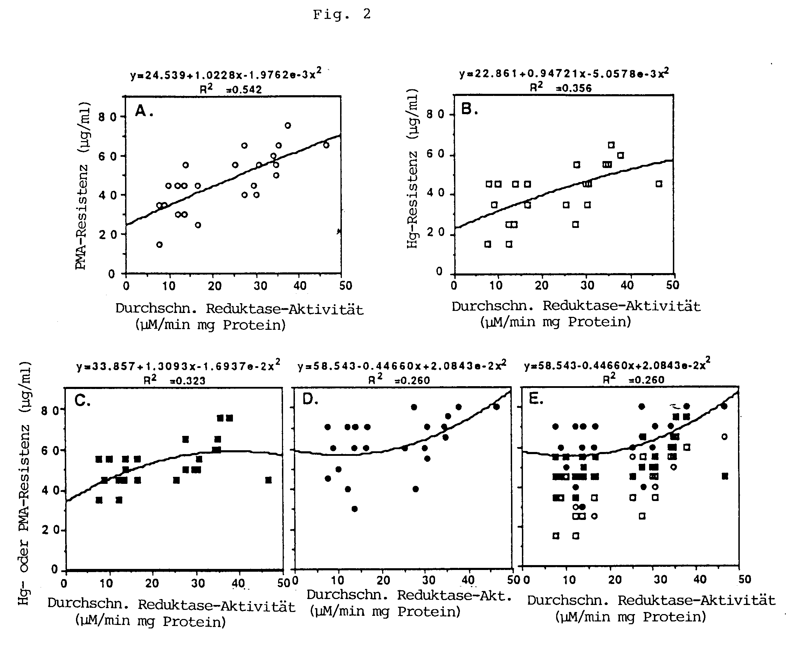

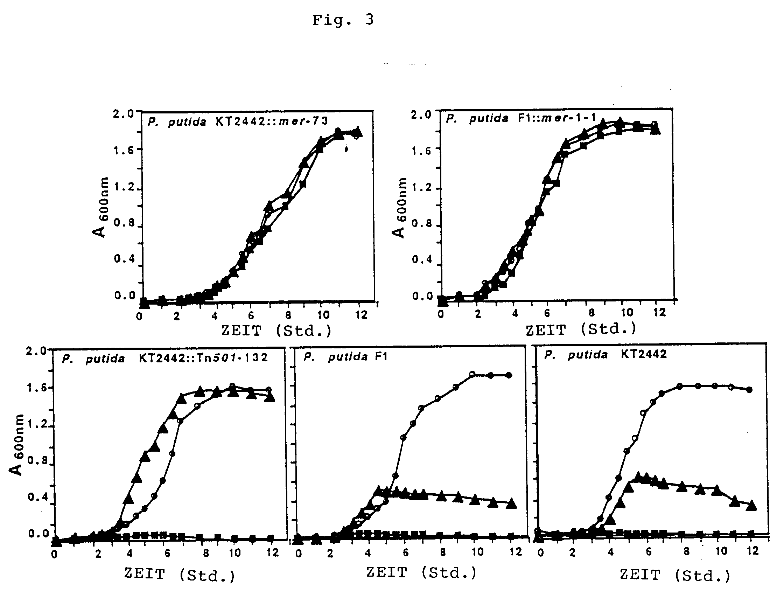

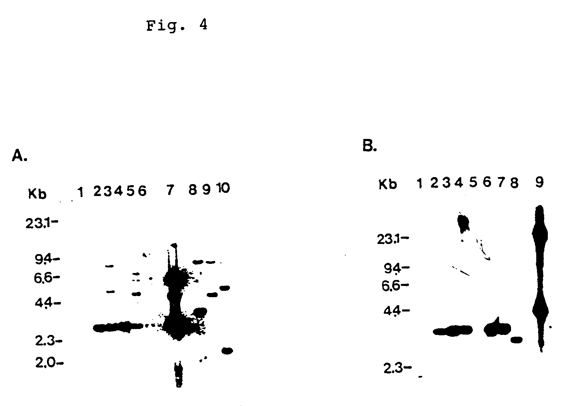

Das Durchtesten einer großen Zahl von P. putida-Mini-Tn5-Akzeptoren nach einer Konjugation zeigte, daß unter den getesteten Isolaten eine große Variationsbreite an PMA-Resistenz existierte. Die weitere Charakterisierung selektionierter, gescreenter Isolate zeigte auch divergierende Werte für Hg$^r$, (Fig. 1), die unterschiedlichen Werten für die Quecksilber-Reduktase-Aktivität entspricht (Tabelle 2, Fig. 2). Die Southern-Blot-Analyse zeigte, daß tatsächlich Traspositionsereignisse stattgefunden hatten, und daß in jedem der getesteten Isolate eine einzelne Kopie von merTPAB vorhanden war (Fig. 4). Deshalb zeigt der Vergleich verschiedener P. putida::mer-Stämme unterschiedliche Werte der merTPAB-Expression, was wahrscheinlich auf unterschiedliche Promotor-Aktivitäten zurückzuführen ist, wobei jedoch eine gründliche Charakterisierung der Transkriptions-Parameter in diesen Isolaten noch aussteht. Die hier vorgestellten Daten stehen deshalb in Einklang dammit, daß die Transkription von merTPAB in den erzeugten, analysierten P. putida::mer-Stämmen zumindest teilweise durch exogene Promotoren des Wirtes erfolgt.

Die Selektion von hyperexprimierenden P. putida::mer-Derivaten wurde durch Screenen auf PMA erreicht, eine Originalvorschrift, die den falsch positiven Hintergrund unterdrückt, der beim Screenen auf HgCl$_2$ auftrat. mer-Hyperexprimierende P. putida-Abkömmlinge erwiesen sich als deutlich stärker Hgr/PMA$^r$, eine Erkenntnis, die nicht nur ihre Selektion ermöglichte, sondern auch neu ist, da die gleichen Effekte in E. coli nicht hervorgerufen werden konnten (23, 25, 31).

Die Erfinder untersuchten annähernd 2000 mit mini-Tn5-mer mutagenisierte Derivate von P. putida KT2442 und F1. Die folgenden Stämme wurden bei der Deutschen Sammlung für Mikroorganismen hinterlegt:

P. putida KT 2442 :: mer-73 DSH 7209
P. putida F1 :: mer-1-1 DSM 7208

Der Wahrscheinlichkeit nach sollte bei der gegebenen Anzahl von getesteten Isolaten unter den verwendeten Bedingungen der höchste erreichbare Wert für die PMA$^r$ aufgefunden worden sein. Wenn in vivo durch die Einschränkungen des Hg(II)-Transports höhere Werte an Resistenz gefunden werden, können diese in P. putida durch das hier beschriebene Verfahren definiert worden sein. Ein alternativer Versuch zur Erreichung noch höherer Hg(II)-Reduktion in vivo könnte sein, die Transport-Determinanten, merTP, unter definierte, aber von merAB getrennte Regulationskontrolle zu bringen. merAB codiert für das (Organo-)Quecksilber transformierende Funktionen.

Konstitutive Hyperexpression von merTPAB erzeugt offensichtlich unmittelbare Hg$^r$, sogar unter toxischen Konzentrationen von Hg(II), und es wurden keine Auswirkungen der mer-Hyperexpression auf die Wachstumsraten der Kulturen gefunden (Fig. 3). Durch Vereinigen der konstitutiven Hyperexpression von merTPAD mit der Kapazität von P. putida F1, Benzol abzubauen, wurde sowohl Wachstum als auch Entgiftung von PMA erreicht, wenn dieses als einzige Kohlenstoff- und Energiequelle eingesetzt wurde.

Die vorliegend beschriebenen Isolate von P. putida::mer weisen vorteilhafte Eigenschaften für die Entgiftung von Hg-haltigen Abfällen sowohl in Behältern als auch in der freien Umwelt auf. Da sich die Lösungsstrategie der vorliegenden Erfindung auf enzymatische Reduktion stützt, ist sie spezifisch für (gebildetes) Hg(II) und vermeidet die unselektiven Eigenschaften derzeit gängiger adsorptiver Systeme.

Die vorliegend beschriebenen Stämme sind in der Lage, in Gegenwart hochkonzentrierter Quecksilberverbindungen zu wachsen und diese Fähigkeit konstitutiv zu exprimieren; der Zelltod in hochbelasteten Abfällen vor der vollständigen Induktion der Expression wird verhindert. Ferner ist unter Bedingungen, in denen die Hg(II)-Konzentrationen in der Umgebung unterhalb der Toxizität liegen und zu niedrig sind, um in eingesessenen Populationen Hg(II)-Reduktion zu induzieren, eine konstitutive Bioentgiftung ebenfalls von Vorteil.

P. putida ist ein in der Natur vorkommender, den Boden besiedelnder Organismus, der beinahe ubiquitär verteilt ist. Deshalb sollten P. putida::mer-Stämme weniger Risiken bezüglich der Aufrechterhaltung des natürlichen ökologischen Gleichgewichts bergen, wenn sie absichtlich als Mittel zum Unschädlichmachen von (Organo-)Quecksilber freigesetzt würden. Die genetische Analyse zeigt auch, daß eine einzelne Kopie des mer-Operons stabil in das Wirtschromosom eingebaut ist (Fig. 4); das Risiko der Übertragung von merTPAB auf natürlich in der entsprechenden Umgebung vorkommende Organismen sollte deshalb deutlich reduziert sein.

P. putida::mer-Isolate entgiften sowohl Hg-Salze als auch Organoquecksilberverbindungen, und P. putida F1::mer-Abkömmlinge sind fähig, Benzol- und Toluol-Quecksilberderivate als einzige Kohlenstoffquelle zu nutzen. Dies gibt die Möglichkeit, Mischungen von Hg-haltigen Verbindungen wie auch Benzol und Toluol, die ihrerseits toxisch sind, zu entgiften. Die Notwendigkeit, dem System von außen Nährstoffe zuzuführen, könnte sich ebenfalls erübrigen, während gleichzeitig eine Toxizität für die Zelle durch eine Akkumulierung von aromatischen Endprodukten vermieden wird.

Durch Manipulation der natürlich auftretenden bakteriellen Systeme für die Entgiftung von Quecksilberverbindungen werden verbesserte Möglichkeiten und Mittel zur Behandlung von quecksilberhaltigen Abfallströmen und kontaminierten Orten zur Verfügung gestellt.

Die Fähigkeit zur Hg-Transformation ist mittels der Atomabsorptionsspektroskopie, on-line, ermittelt worden. Dabei ist eine im Vergleich zu P.putida KT2442::Tn501-132. 10-fach verbesserte Umsatzgeschwindigkeit festgestellt worden.

Die Erfindung soll im folgenden anhand von Figuren, Tabellen und Beispielen näher erläutert werden.

Die Figuren zeigen im einzelnen:

In Fig.1 ist die maximale Resistenz von P. putida::mer-Isolaten gegenüber $HgCl_2$ und PMA aufgetragen. Dabei handelt es sich um die höchsten Konzentrationen an PMA in festem (■) bzw. flüssigem (▨) und an $HgCl_2$ in festem (▨) bzw. flüssigem (▨) LP121-Medium, bei denen Wachstum der P. putida::mer-Stämme noch möglich war. 10 bzw. 15 $\mu$g/ml PMA in flüssigem bzw. festem LP121-Medium inhibierten das Wachstum von P. putida KT2442::Tn501-132 und der elterlichen Stämme P. putida KT2442 und F1. Das Wachstum der elterlichen Stämme wurde auch durch $HgCl_2$ in Konzentrationen von 6 bzw. 35 $\mu$g/ml in flüssigem bzw. festem LP121-Medium inhibiert.

Fig.2 zeigt die Korrelation der PMA- bzw. $HgCl_2$-Resistenz mit der merTPAD-Expression. Dabei wurde die gemittelte Quecksilber-Reduktase-Aktivität (in Gegenwart von bzw. ohne Hg, Tabelle 2) von 22 untersuchten Isolaten als Funktion ihrer maximalen PMA-Resistenz, beobachtet in flüssigem (○) oder festem (●) LP121-Medium, bzw. als Funktion ihrer maximalen Hg-Resistenz in flüssigem (□) oder festem LP121-Medium (■) dargestellt. Oberhalb einer jeden graphischen Darstellung sind quadratische Gleichungen für die dargestellten Graphen mit den entsprechenden Korrelations-Koeffizienten ($R^2$) angegeben. Der F-Wert, eine statistische Determinante für die Anpassung der Daten an die berechneten Graphen, wurde für die Daten der graphischen Darstellungen A, B, C, D bzw. E mit 8,74, 3,05, 2,45, 1,52 bzw. 6,53 berechnet. Werte von mehr als 4,32 für F (Darstellungen A, B, C und D) oder 3,96 (Darstellung E) zeigen eine signifikante (95% Sicherheit) Korrelation zwischen den Daten und der gezeigten Funktion.

Fig.3 zeigt das Wachstum von P. putida-Stämmen in Gegenwart von $HgCl_2$. LP121-Medien mit 6 $\mu$g/ml $HgCl_2$ (■), mit 6 ug/ml $HgCl_2$, zugesetzt bei $A_{600} = 0,5$ (▲), oder ohne den Zusatz von $HgCl_2$ (○) wurden mit einer Kultur in stationärer Phase beimpft (1:100) und bei 30°C inkubiert. P. putida::Tn501-132 wurde vor der Zugabe von $HgCl_2$ in der midlog-Phase mit 10 $\mu$m $HgCl_2$ 2 Stunden lang voriduziert.

Fig. 4 zeigt eine Hybridisierungs-Analyse nach Southern von P. putida::mer-Stämmen. Die Blots wurden mit einem aus pUTHg stammenden, merTPAB enthaltenden markierten Fragment (3,2 kB) als Sonde versehen. Die Spuren der Abb. (A) zeigen P. putida::mer KT2442-Stämme: 1: P. putida::mer KT2442 (SfiI); 2: -67 (SfiI); 3: -73 (SfiI);, 4: -2-4 (SfiI); 5: -2-49 (SfiI); 6: -2-121 (SfiI); 7: pUTHg (SfiI); 8: -67 (EcoRI); 9: -73 (EcoRI); 10: -2-4(EcoRI). Die Spuren der Abb. (B) zeigen P. putida::mer F1-Stämme: 1: P. putida::mer F1 (SfiI); 2: -1-1 (SfiI); 3: -1-7 (SfiI); 4: pUTHg (SfiI); 5: -1-22 (SfiI); 6: -10 (SfiI), 7: -13 (SfiI); 8: P. putida::mer KT2442::Tn501-132 (Aval); 9: pUTHg (EcoRI).

## Tabelle 1. Bakterien und Plasmide

| Stamm oder Plasmid | Beschreibung | Quellenangabe |
|---|---|---|
| **Bakterien** | | |
| *Escherichia coli* | | |
| HB101 | *proA2 leuB6 thi-1 lacY1 hsdR hsdM recA13 supE44 rpsL20* | 5 |
| C600 | *supE44 hsdR thi-1 thr-1 leuB6 lacY1 tonA21* | 40 |
| SM10($\lambda pir$) | *thi-1 thr leu tonA lacY supE recA*::RP4-2-Tc::Mu, Km[r], $\lambda pir$ | 20 |
| *Pseudomonas putida* | | |
| KT2442 | *hsdR*, Rif[r] | 18 |
| F1 | *todFC1C2BADEJ*[+], Tol[+]Ben[+] | 10 |
| KT2442::Tn*501*-132 | *hsdR merTPAD*[+], Hg[r]Rif[r] | 9a |
| KT2442:*mer* | *hsdR merTPAB*[+], PMA[r]Hg[r]Rif[r] | Erfindung |
| F1::*mer* | *todFC1C2BADEJ*[+] *merTPAB*[+], PMA[r]Hg[r]Tol[+]Ben[+] | Erfindung |
| **Plasmide** | | |
| pRK2013 | *ori* ColE1[+] *tra* RK2[+], Km[r] | 6 |
| pFRC37p | *ori* ColE1[+] *mob* RP4[+] Tn*501 merR*[+] *merTPAD*[+], Ap[r] | F. Rojo |
| pUTHg | *ori* R6K[+] *mob* RP4[+] *merTPAB*[+]$\Delta$*merDR*, Ap[r] Hg[r] | 12 |
| pGP704 | *ori*R6K *mob*RP4, MCS of M13tg131, Ap[r] | 20 |

EP 0 585 652 A2

Tabelle 2.  Quecksilber-Reduktase-Aktivitäten
von mit $HgCl_2$ induzierten oder uninduzierten
P. putida::mer-Kulturen

| Stamm/Isolat | Quecksilber-Reduktase-Aktivität[a] | |
|---|---|---|
| | - $HgCl_2$ | + $HgCl_2$ |
| *P. putida* KT2442 | 0 | ND[b] |
| *P. putida* F1 | 0.2 | ND |
| *P. putida* KT2442::Tn*501*-132 | 0 | 12.1 |
| *P. putida* KT2442::*mer* | | |
| 37 | 7.0 | 8.1 |
| 45 | 14.5 | 12.7 |
| 67 | 26.0 | 29.0 |
| 73 | 50.8 | 45.7 |
| 2-1 | 9.7 | 8.2 |
| 2-3 | 14.3 | 18.7 |
| 2-4 | 29.0 | 32.4 |
| 2-49 | 27.3 | 23.4 |
| 2-65 | 12.7 | 11.6 |
| 2-84 | 12.7 | 15.1 |
| 2-116 | 15.9 | 17.3 |
| 2-121 | 28.7 | 31.0 |
| *P. putida* F1::*mer* | | |
| 10 | 34.2 | 35.4 |
| 12 | 7.7 | 7.9 |
| 13 | 33.3 | 36.3 |
| 18 | 10.1 | 9.8 |
| 31 | 29.4 | 31.1 |
| 38 | 12.8 | 11.5 |
| 1-1 | 35.8 | 39.6 |
| 1-6 | 13.3 | 14.2 |
| 1-7 | 35.0 | 35.9 |
| 1-14 | 27.1 | 28.2 |
| 1-22 | 34.0 | 34.6 |

[a]Die Quecksilber-Reduktase-Aktivitäten sind ausgedrückt als $HgCl_2$-abhängige NADPH-Oxidation (µM oxidiertes NADPH/min mg Protein) wie in Beispiel 1 beschrieben

[b]ND = nicht bestimmt

Tabelle 3. Wachstum und Katechol-2,3-dioxygenase-Aktivität von P. putida Fl::mer-Isolaten auf Minimalmedien mit PMA als einziger Kohlenstoffquelle

| Stamm/Isolat | M63 PMA[a] | |
|---|---|---|
| | Wachstum[b] | Dioxygenase[c] |
| *P. putida* F1 | - | - |
| *P. putida* KT2442::*mer*-73 | - | - |
| *P. putida* F1::*mer* | | |
| 1 0 | + | + |
| 1 3 | + | + |
| 1 8 | + | + |
| 3 1 | + | + |
| 1 - 1 | + | + |
| 1 - 6 | + | + |
| 1 - 7 | + | + |
| 1 - 1 4 | + | + |

[a]Die M63-Salze wurden durch 160 µg/ml PMA als einzige Kohlenstoffquelle ergänzt

[b]Wachstum isolierter Kolonien nach dreitägigem Wachstum bei 30°C: (+) weist auf sichtbare Kolonien, (-) bedeutet fehlendes Wachstum

[c]Positive Aktivität von Katechol-2,3-dioxygenase (+) gemäß Farbtest nach Ansprühen der Kolonien mit 0,5 M Katechol (9). (-)= keine wahrnehmbare Aktivität

Beispiele

Beispiel 1. Materialien und Methoden

(a) Bakterienstämme, Plasmide und Wachstumsmedien

Die in der vorliegenden Erfindung verwendeten Bakterienstämme und Plasmide sind in Tabelle 1 dargestellt. L-Medium enthielt 1 % Trypton (Difco), 0,5 % Hefeextrakt (Difco) und 0,5 % NaCl. M9- und M63-Minimalmedien (19) wurden jeweils durch 0,2 % Citrat als Kohlenstoffquelle ergänzt. 0,001 % Pseudomonas-Stammsalze (1) ergänzten zusätzlich das Minimal-M9-Medium; 0,1 % Casaminosäuren (Difco) wurden zum M63-Minimalmedium gegeben. 121-Salze-Minimalmedium mit geringem Phosphatgehalt (100 uM) (LP121) wurde wie bereits beschrieben (14) hergestellt. Die Medien wurden mit 1,5 % Agar (Difco) verfestigt. In Selektionsmedien wurden Antibiotika in den folgenden Konzentrationen (pro ml) verwendet: Rifampicin (Rif): 50 µg; Kanamycin (Km): 30 µg; Ampicillin (Ap): 50 µg; Carbenicillin (Cb): 500 µg; Piperacillin (Pc): 30 µg. Hg(II), in Form von $HgCl_2$ (Sigma) aus einer Vorratslösung von 10 mg/ml, wurde in den angegebenen Konzentrationen zu den vorgekühlten Medien gegeben. Konzentrierte Vorratslö-

8

sungen von Phenylquecksilberacetat (PMA) (Sigma) (1 mg/ml) wurden 2 Stunden lang bei 30°C heftig geschüttelt, bevor sie den Medien in den angegebenen Konzentrationen zugesetzt wurden.

(b) Mobilisierung und Transposition

Das transposonhaltige Plasmid ist aus Escherichia coli SM10 lambda pir (pUT-Hg) durch Mobilisierung mit einer biparentalen Filter-Mating-Technik in entweder P. putida KT2442- oder P. putida F1-Recipienten transferiert worden (6). Einzelne Kulturen der Donoren und der Akzeptoren wurden bei 30 °C in L-Medium, das durch selektive Antibiotika ergänzt war, bis zur stationären Phase gezüchtet. 0,4 ml des Akzeptor-Stammes und 0,1 ml eines jeden Donors wurden in 5 ml L-Medium vermischt und dann filtriert (0,2 u Swinnex). Die Filter wurden mit der Zellseite nach oben auf die Oberfläche von L-Medium-Platten gegeben und bei 37°C 9 h lang inkubiert. Die Konjugationsprodukte wurden dann in 5 ml 0,85-%iger Salzlösung resuspendiert, und geeignete Verdünnungen wurden auf selektiven Medien ausplattiert. M9-Medium, supplementiert mit Rif und 6 $\mu$g/ml HgCl$_2$, wurde verwendet, um auf pFRC37p-Mobilisierung und Tn501-Transposition in P. putida KT2442 zu selektionieren. M63-Medium mit Rif und 1,5 $\mu$g/ml HgCl$_2$ wurde verwendet, um auf pUTHg-Mobilisierung und mini-Tn5-Transposition in P. putida KT2442 zu selektionieren. M63-Medium, nur durch 1,5 $\mu$g/ml HgCl$_2$ supplementiert, wurde für die Selektion auf die Mobilisierung von pUTHg und die mini-Tn5-Transposition in P. putida F1 verwendet. Selektive Platten wurden bei 30°C inkubiert. Die Exkonjugantien wurden entweder auf M9-Medium mit Rif und 6 $\mu$g/ml HgCl$_2$ auf die Paarung C600 (pFRC37p) * P. putida KT2442 oder auf M63-Medium, ergänzt durch 10 $\mu$g/ml PMA, auf die Akzeptoren der pUT-Hg-Paarungsergebnisse durchgetestet./Die gescreenten Konjugationsprodukte wurden auch daraufhin untersucht, ob sie, soweit es sich um P. putida KT2442 handelte, gegenüber Rif resistent waren (Rif$^r$), bzw. ob sie gegenüber Cb, Pc und Km empfindlich waren (Cb$^s$, Pc$^s$ bzw. Km$^s$).

(c) Bestimmung von erhöhten Werten der Hg-Resistenz.

Die Konjugationsprodukte der P. putida-Stämme wurden von L-Medium-Platten mit 10 $\mu$g/ml PMA auf L-Medium-Platten mit 50 bis 250 $\mu$g/ml PMA replikaplattiert. Diejenigen Stämme, die bei erhöhten PMA-Konzentrationen Wachstum isolierter Kolonien aufwiesen, wurden weiterhin auf das Wachstum isolierter Kolonien auf LP121-Platten untersucht, die entweder 1,5 bis 60 $\mu$g/ml HgCl$_2$ oder 10 bis 75 $\mu$g/ml PMA enthielten. Die Stämme von P. putida wurden auf die oberen Grenzwerte von Hg$^r$ in flüssigem Medium hin untersucht, indem die Kulturen isolierter Konjugationsprodukte bei 30°C in LP121-Medium bis zur mid-log-Phase ($A_{600} = 0,5$) gezüchtet wurden, worauf ihnen HgCl$_2$ oder PMA entsprechend der Endkonzentration zugesetzt wurde (alternativ wurden die pFRC37p-Exkonjugantien bei $A_{600} = 0,5$ mit 0,8 $\mu$g/ml HgCl$_2$ induziert und dann zwei Stunden lang vorinkubiert, worauf ihnen HgCl$_2$ bis zur Endkonzentration zugesetzt wurde). Die $A_{600}$ wurde nach etwa 4 Stunden Wachstum in HgCl$_2$ oder PMA bestimmt und mit der $A_{600}$ paralleler Kulturen verglichen, die unter den gleichen Bedingungen, jedoch ohne Zusatz von HgCl$_2$ bzw. PMA, gezüchtet worden waren.

(d) Bestimmung der Wachstumskurven

Kulturen stationärer Phase von P. putida-Stämmen wurden in LP121-Minimalmedium angeimpft (1:100). Parallele Kulturen, die bei 30°C ohne HgCl$_2$ oder mit einer Anfangskonzentration von 6 $\mu$g/ml HgCl$_2$ oder mit einem Zusatz von 6 $\mu$g/ml HgCl$_2$ nach Erreichen der mid-log-Phase (Tn501-haltiger Stamm wurde eine Stunde lang mit 2,7 $\mu$g/ml HgCl$_2$ vorinduziert, bevor 6 $\mu$g/ml zur midlog zugesetzt wurden) gezogen worden waren, wurden in regelmäßigen Abständen durch Bestimmung der $A_{600}$ überwacht. Auszählen von Platten mit Lebendkulturen wurde verwendet, um die Ergebnisse zu sichern.

(e) Quecksilber-Reduktase-Bestimmungen

Die Zellen wurden bei 30°C in LP121-Kulturen von 50 ml bis zu $A_{600} = 0,5$ gezüchtet, worin man sie entweder ohne Zusatz von HgCl$_2$ weiterwachsen ließ, oder worauf HgCl$_2$ in der höchsten Konzentration zugesetzt wurde, die Wachstum noch zuließ (wenn es sich um Konjugationsprodukte (Exkonjugantenstämme) von pUTHg handelte), oder worauf sie 2 Stunden lang mit 0,8 $\mu$g/ml HgCl$_2$ induziert wurden (Tn501-haltiger Stamm) und dann mit HgCl$_2$ auf 6 $\mu$g/ml versetzt wurden. Die Kulturen wurden bis zu einem Endwert von $A_{600} = 1,5$ gezüchtet und dann durch Zentrifugation geerntet (10.500 x g, 10 min., 4°C), einmal mit 20 ml kaltem 100 mM NaPO$_4$ (pH 7,5), 0,5 mM EDTA (Fluka), 0,1 % $\beta$-Mercaptoethanol (Sigma) gewaschen und in 1 ml des gleichen Puffers resuspendiert. Die Zellen wurden mit Ultraschall lysiert (50W, 2 min., B. Braun Labsonic U), und die unlösliche Fraktion wurde abzentrifugiert (27.000 x g, 4°C, 30 min.). Der Überstand wurde mit Hilfe einer Modifikation des Verfahrens von Fox und Walsh (8) und Schottel (36) auf Quecksilber-Reduktase getestet. Hierzu sei kurz ausgeführt, daß 0,2 ml Rohextrakt zu einer Testmischung gegeben wurden, die Endkonzentrationen von 50 mM $NaPO_4$ (pH 7,5), 200 uM NADPH (Sigma), 1 mM $\beta$-Mercaptoethanol und 100 uM $HgCl_2$ in einem Reaktionsvolumen von insgesamt 3 ml enthielt. Die Umsetzungen wurden 4 Minuten lang bei 25°C durchgeführt, und während dieser Zeit wurde die Oxidation von NADPH spektrophotometrisch bei 340 nm verfolgt; die Hintergrundwerte der NADPH-Oxidation wurden bestimmt, indem Parallelumsetzungen ohne $HgCl_2$ beobachtet wurden. Die Proteinkonzentrationen wurden mit Hilfe der BCA-Bestimmung (Pierce) ermittelt, durchgeführt gemäß den Empfehlungen des Herstellers.

(f) Southern Blot

Genomische DNA wurde nach dem Verfahren von Richards (33) isoliert; Plasmid-DNA wurde durch alkalische Lyse isoliert und durch CsCl-Gleichgewichts-Zentrifugation gereinigt (35). 10 ug genomischer DNA wurden entweder mit AvaI oder SfiI oder EcorI verdaut, und 0,1 ug der Plasmid-DNA wurden mit SfiI (Boehringer Mannheim) gemäß den Angaben des Herstellers geschnitten. Verdaute DNA-Proben wurden über ein 0,7 % Agarose-Plattengel (35) laufen gelassen. Die Gele wurden auf Biodyne "B"-Membranen (Pall Biosupport) geblottet, und die Membranen wurden mit markierten DNA-Sonden gemäß den Vorschlägen des Herstellers hybridisiert. Die DNA-Fragmente, die als Sonden eingesetzt wurden, wurden unter Verwendung von Gene Clean (Bio 101, Inc.,) von Agarose-Plattengelen isoliert; als Sonde eingesetzte Fragment- wie auch Plasmid-DNA wurden in vitro mit [alpha-$^{32}$P]-dCTP (Amersham) markiert, und zwar unter Verwendung zufälliger Primer-Verlängerung ("random primer extension") (Multiprime DNA-Labeling System, Amersham).

Beispiel 2. Konstruktion, Selektion und Screening von P. putida-Derivaten mit hoher Resistenz gegenüber PMA (PMA$^r$).

Theoretisch könnte die Hyperexpression von Determinanten mit breitem Wirkungsspektrum zu erhöhten Resistenzwerten gegenüber Organoquecksilberverbindungen und gleichzeitig einem Anstieg der Entgiftungseigenschaften der entstandenen Stämme gegenüber (Organo-)Quecksilberverbindungen führen.

Um eine Überproduktion der mer-Genprodukte zu erzielen, wurde erfindungsgemäß das Plasmid pUTHg eingesetzt, das ein Replikon mit enger Wirtsspezifität ist und für die merTPAB-Quecksilber-Resistenz mit breitem Wirkungsspektrum, von pDU1358 stammend, codiert (11). Die pUTHg-merTPAB-Gene werden von den invertierten Wiederholungssequenzen von Tn5 flankiert, die auf 19 Basenpaare (Bp) verkürzt sind. Das Transposase-Gen (tnp*) ist auf pUTHg bezüglich des transponierenden Elementes von Tn5 extern angeordnet, und merR und merD, die für mer-Regulatorproteine codieren, wurden aus dem Konstrukt eliminiert (siehe unten, 11, 12).

Da vorangehende Studien gezeigt haben, daß ein Anstieg der mer-Gen-Dosis in Escherichia coli nicht gleichermaßen zu einem Anstieg der Quecksilber-Resistenz führte (23, 25), wurden zwei Stämme von P. putida als Akzeptoren für pUTHg in biparentalen Matings ausgewählt. P. putida F1 ist ein in der Natur vorkommendes, den Boden besiedelndes Isolat (10), während P. putida KT 2442 ein gegenüber Rifampicin resistenter Abkömmling eines isolierten Bodenorganismus' ist (18). Bodenisolate wurden primär wegen ihrer potentiellen Verwendbarkeit für die Sanierung von mit Quecksilber verseuchten Böden und Wässern verwendet. Beide Stämme sind bereits in der Vergangenheit charakterisiert worden, und F1 beinhaltet einen durch seine Chromosomen codierten Pathway für den Katabolismus von Benzol (10). Der Benzolabbau könnte also in diesem Organismus mit der Entgiftung von Quecksilber gekoppelt werden.

Nach dem "Mating". also dem "Paaren" der Akzeptoren P. putida KT2442 bzw. F1 mit dem geeigneten Donorstamm unter Konjugationsbedingungen, konnte man feststellen, daß die Transpositionsfrequenzen für die merTPAB-Gene in sinnvoller Weise mit früher beobachteten (12) übereinstimmten. Um weitere Screenings zu erleichtern und um die Resistenz gegenüber Organoquecksilber-Verbindungen zu sichern, wurden 1025 Isolate aus der Konjugation von P. putida KT2442 und 800 aus der von P. putida F1 auf mit 10 $\mu$g/ml PMA ergänztes L-Medium übertragen.

Da angenommen wurde, daß die Mini-Tn5-merTPAB-Transposition bezüglich der Zielorte im allgemeinen ungerichtet verläuft, wurde der Schluß gezogen, daß Insertionen, die strangabwärts zu proximalen Wirts-Promotoren gelegen sind, zu einem Anstieg der mer-Expression führen könnten, wobei gleichzeitig die Hg- und PMA-Resistenz (Hg$^r$, PMA$^r$) ansteigen sollte. Deshalb wurde die weitere Selektion der Akzeptor-Isolate mit erhöhter Hg$^r$/PMA$^r$ durchgeführt, indem diese Produkte der Konjugation von P. putida KT2442 und F1 auf quecksilber(II)-haltigem L-Medium gescreent wurden. Da festgestellt wurde, daß bei hohen Zelldichten häufig auch die Hg-empfindlichen (Hg$^s$) Stämme auf HgCl$_2$-haltigen Medien wachsen, wurde den L-Medien bevorzugt das wenig flüchtige Phenylquecksilberacetat (von 50 bis 250 $\mu$g/ml) zugesetzt. Durch dieses Verfahren wird die Selektion von falsch-positiven Klonen vermieden; die resistenten Phänotypen lassen sich klar von den sensitiven unterscheiden.

Unterschiedliches Wachstum der P. putida KT2442xpUTHg-Exkonjugantien wurde auf Medien beobachtet, die 50 bis 80 $\mu$g/ml PMA enthielten, während die P. putida F1xpUTHg-Isolate unterschiedliches Wachstum auf Medien zeigten, die 50 bis 250 $\mu$g/ml PMA enthielten. Oberhalb dieser Obergrenzen der PMA-Konzentration wuchs keines der getesteten Isolate. Einzelne repräsentative Konjugationsprodukt-Stämme (20 P. putida KT2442 x pUTHg und 20 P. putida F1 x pUTHg), die Wachstum isolierter Kolonien auf PMA-haltigem L-Medium zeigten, wurden für weitere Untersuchungen ausgewählt.

Diese 40 Exkonjugantien-Stämme wurden auf ihre Fähigkeit getestet, auf Cb, Pc oder Km enthaltenden L-Medien zu wachsen, und alle Stämme erwiesen sich als empfindlich gegenüber allen drei Antibiotika (zusätzlich erwiesen sich P putida KT2442-Derivate als resistent gegenüber Rif ), wodurch sichergestellt war, daß keine Replikation oder Integration eines der Donorplasmide in die untersuchten Akzeptoren stattgefunden hatte. Es konnte also gezeigt werden, daß alle Stämme, die weiteren Untersuchungen unterzogen wurden, einer Transposition von mini-Tn5 in das Wirts-Chromosom (::mer) unterworfen worden waren.

Beispiel 3. Bestimmung der Obergrenzen der Resistenz gegenüber HgCl$_2$ und PMA in Minimal-Medien

Die Höchstwerte der Konzentration an HgCl$_2$ und PMA, die noch ein Wachstum in sowohl festem als auch flüssigem LP121 erlaubten, wurden für die 40 zuvor untersuchten P. putida::mer-Stämme bestimmt. Für diese Experimente wurde P. putida KT2442::Tn501-132, das eine stabile Insertion des für ein mer-Operon mit geringem Wirkungsspektrum codierenden Tn501 (3, 38) enthält, durch Mobilisierung von pFRC37p konstruiert (siehe Beispiel 1). P. putida KT2442::Tn501-132 lieferte einen Vergleichswert für prototypische Hg$^r$ und Expression durch einen nativen mer-Promotor.

P. putida::mer-Stämme, die Wachstum isolierter Kolonien auf LP121-Agar mit HgCl$_2$ oder PMA zeigten, wurden als resistent gegenüber der zugefügten Konzentration an Quecksilberverbindung eingestuft. Sie wurden dann auf die gleiche Weise bei höheren Konzentrationen an HgCl$_2$ und PMA weitergetestet, bis die Werte erreicht waren, die das Wachstum isolierter Kolonien inhibierten.

Die Höchstwerte der Resistenz in flüssigen LP121-Medien wurden bestimmt, indem Kulturen unter aeroben Bedingungen bis zum Erreichen der midlog-Wachstumsphase (A$_{600}$ = 0,5) inkubiert wurden und an diesem Punkt die Quecksilberverbindung zugesetzt wurde. Das spektroskopische Absorptionsvermögen wurde für jede Kultur nochmals nach vierstündigem kontinuierlichem Wachstum ermittelt. Die Differenzen der optischen Dichten wurden berechnet und mit den Differenzen des Absorptionsvermögens paralleler, unbehandelter Kulturen verglichen, die zu denselben Zeitpunkten gemessen worden waren. Da Empfindlichkeit gegenüber HgCl$_2$ oder PMA einer gegebenen Konzentration zu einem vernachlässigbaren Wachstumsanstieg führte (Daten nicht gezeigt), wurde als Resistenz definiert, daß annähernd die optische Dichte der unbehandelten Kontrolle erreicht wurde. Repräsentative Ergebnisse dieser Experimente sind in Fig. 1 dargestellt.

85 % der 20 getesteten P. puitda KT2442::mer-Isolate zeigten einen 12,5-37,5-%igen Anstieg des Hg$^r$-Wertes im Vergleich zu P. putida KT2442::Tn501-132, wenn sie auf festen LP121-Medien gezüchtet wurden. Nur 30 % derselben Stämme zeigen jedoch einen Anstieg an Hg$^r$, wenn man sie in flüssigem LP121 wachsen läßt; die Erhöhung lag in diesen Fällen jeweils um 28,5 % über den Werten von P. putida KT2442::Tn501-132. Jedoch zeigten ganz allgemein diejenigen P. putida KT2442::mer-Stämme die größte demonstrierbare Resistenz beim Wachstum in Flüssikeit, die die höchste Hg$^r$ auf festen Medien aufwiesen. Wurden diese 20 P. putida KT2442::mer-Isolate auf PMA-haltigen LP121-Medien getestet, zeigten sie höhere Resistenz über einen kürzeren Bereich an PMA-Konzentrationen auf festen Medien, als wenn sie in flüssigen Medien gezüchtet wurden.

Im allgemeinen zeigten die 20 P. putida F1::mer-Stämme eine größere Hg$^r$ als die untersuchten P. putida KT2442::mer. 50 % wiesen auf festen bzw. flüssigen LP121-Medien einen 14,3-85,7-%igen Anstieg an Hg$^r$ im Vergleich zu P. putida KT2442::Tn501-132 auf. Dagegen waren die Obergrenzen für PMA$^r$ zwischen den mer-Abkömmlingen von P. putida KT2442 und F1 vergleichbar (Fig. 1).

Distal zu den merA-Genen von Tn501 gelegene DNA-Sequenzen und die Plasmide R100 und pDU1358 sind von Bedeutung für die Hg$^r$ und weisen einen hohen Komologiegrad auf (4, 11, 13, 22, 25, 29). Die Deletion dieser Sequenzen, die für mindestens zwei offene Leserahmen codieren (merD und urf1), vermindert die Höhe der maximalen Hg$^r$ um 20 % (4, 22, 25, 29), was mit einer Abnahme der Induzierung der Hg-Volatilisierung (4) und mit einem gleichzeitigen Anstieg der mer-Transkription korreliert (22, 29). Es konnte gezeigt werden, daß das merD-Protein spezifisch an den mer-Promotor ($P_{mer}$) bindet (22). Deshalb ist vorgeschlagen worden, daß MerD als Coropressor der merTPABD-Expression fungiert (22, 29).

Die zum Promotor distal gelegene EcoRV-Stelle, die zur Isolierung des merTPAB-haltigen Fragments für die Konstruktion von pUTHg verwendet wurde (12), ist strangaufwärts von merD gelegen (11). Die Quecksilber-Resistenz mit breitem Wirkungsspektrum, die von mini-Tn5 codiert wird, weist also eine Deletion von merD auf. Trotz des Verlustes von merD konnte jedoch gefunden werden, daß einige der hier untersuchten P. putida::mer-Abkömmlinge eine größere maximale Hg(II)-/PMA-Resistenz aufweisen als P. putida, das Wildtyp-Tn501 beherbergt (Fig.1). Die Tatsache, daß die Resistenz einiger mini-Tn5-Isolate weit höher war als die bereits früher in merD-Mutanten beobachtete, nämlich 60 uM (22), bestätigt, daß die mer-TPAB-Transkription in diesen Isolaten von starken exogenen Wirtspromotoren an ihren jeweiligen chromosomalen Insertions-Zielorten herrührt.

Beispiel 4. mer-Genexpression in P. putida::mer-Isolaten

Da sich die enzymatische Aktivität des merA-Genprodukts, die Quecksilber-Reduktase, aus rohen Zellextrakten bestimmen läßt (36), wurde diese als Maß für die Genexpression von mer gewählt. Quecksilber-Reduktase-Bestimmungen wurden an Extrakten von 12 repräsentativen P. putida KT2442::mer- und 11 P. putida F1::mer-Isolaten ausgeführt, die mit und ohne zugesetztem $HgCl_2$ in LP121-Medien gezüchtet worden waren. P. putida KT2442::Tn501-132 wurde genauso untersucht, nachdem der Stamm in LP121-Medium mit $HgCl_2$ induziert worden war, um den Vergleichswert für die mer-Expression zu liefern, die vom nativen, von Tn501 codierten mer-Promotor herrührt (16, 21, 28). Die Ergebnisse dieser Experimente sind in Tabelle 2 dargestellt.

Eine Expression von merA in P. putida KT2442::Tn501-132 war ohne Zugabe von $HgCl_2$ nicht erkennbar. Wie bereits früher in Studien mit E. coli-Wirten gefunden wurde, ist durch Tn501 codierte merA-Expression mit Hg(II) induzierbar, wie auch das mer-Operon des Plasmids R100 (39). Die Hg(II)-Induzierbarkeit in diesen Systemen wird durch die Aktivität des MerR-Proteins verursacht, das in Gegenwart von Hg(II) als positiver Regulator für die merTPAD-Expression fungiert, in dessen Abwesenheit jedoch als negativer Regulator (7, 16, 25, 30).

Es wurde jedoch berichtet, daß die vom Wildtyp Tn501 codierte mer-Aktivität in Pseudomonas aeruginosa beinahe konstitutiv ist; in Gegenwart bzw. Abwesenheit von Hg(II) wurde eine nur 30 %ige Differenz an MerA-Aktivität beobachtet (4). Es hat sich jedoch vorliegend gezeigt, daß die Expression des Tn501-Operons in P. putida Hg-abhängig ist: in nicht zuvor Hg(II) ausgesetzten Zellen war keine Quecksilber-Reduktase-Aktivität auffindbar (Tabelle 2), und uninduzierte Kulturen waren Hg[s] (siehe unten, Fig. 3).

Im Gegensatz zu der Induzierbarkeit, die bei dem Tn501 vom Wildtyp enthaltenden P. putida-Stamm gezeigt wurde, wiesen alle getesteten 23 P. putida::mer-Isolate annähernd die gleichen Werte für die Quecksilber-Reduktase-Aktivität auf, unabhängig von der Zugabe oder dem Fehlen von $HgCl_2$. Deshalb ist die merTPAB-Expression in diesen Stämmen konstitutiv; ein Zusatz von Hg(II) ist für die merTPAB-Expression nicht erforderlich (Tabelle 2).

Dem Plasmid pHG106, der Quelle von pDU1358-merTPAB-DNA für die Konstruktion von MiniTn5, fehlt das merR-Gen (11, 12). merR-Mutanten von Tn501 und vom Plasmid R100 zeigen normalerweise mikrokonstitutive merTPAD-Expression (d.h. 10fach über dem normalen reprimierten Wert, aber 100fach unterhalb der normalen induzierten Werte), und sie sind phänotypisch Hg[s](25, 28). Das Wildtyp-pDU1358-mer-Operon (aus dem das hier verwendete mini-Tn5 derivatisiert worden war) ist wie Tn501 und R100 ebenfalls induzierbar, wenn es jedoch eine Deletion von merR aufweist, verleiht es E. coli Hg[r] (11). Die konstitutive mer-Expression, die sich bei den vorliegend untersuchten P. putida::mer-Isolaten erkennen läßt, ist also wahrscheinlich eine Folge des Fehlens einer merR-Determinante.

Der Wert der konstitutiven Quecksilber-Reduktase-Aktivität, den die untersuchten P. putida::mer-Isolate aufwiesen, unterschied sich in signifikanter Weise von der induzierten merA-Aktivität, die P. putida KT2442::Tn501-132 aufweist. P. putida KT2442::mer-Isolate zeigten einen bis zu 4,2-fachen Anstieg (Isolat 73), und die Stämme P. putida F1::mer zeigten eine maximal 3,3-fach (Isolat 1-1) höhere Reduktase-Aktivität als der Tn501 enthaltende Stamm. Die Deletion von merD, dem vermutlichen merTPAB-Corepressor, könnte zum Anstieg der mer-Expression beitragen, der in diesen P. putida::mer-Isolaten beobachtet wurde. Die Tatsache jedoch, daß die Expression zwischen ::mer-Isolaten stark schwankt (siehe Tabelle 2), stützt wiederum die Vermutung, daß die Transkription in diesen Stämmen mindestens teilweise von exogenen Wirtspromotoren an den Orten der mer-Insertion ausgelöst wird.

### Beispiel 5. Beziehung zwischen PMA$^r$ und Hg$^r$ und der merTPAB-Expression

Die Quecksilber-Reduktase-Aktivitäten der jeweils untersuchten P. putida::mer-Isolate wurden gegen die Resistenz derselben Stämme gegenüber PMA oder Hg(II) in festen oder flüssigen LP121-Medien aufgetragen. Die Ergebnisse dieser Analyse sind in Fig. 2 dargestellt. Die Daten erfüllen im großen und ganzen eine quadratische Funktion, was zeigt, daß die Resistenz mit dem Quadrat (bzw. einem Anteil davon) der Reduktase-Aktivität steigt. Die in flüssigen LP121-Medien gemessenen Resistenzen zeigten bessere Korrelation zur mer-Expression als diejenigen, die auf festen Medien bestimmt worden waren. Bei der Transformation aller Korrelationen in statistische Zahlenpaare fielen jedoch nur die aus den Resistenzbestimmungen in flüssigen, PMA enthaltenden LP121-Medien gewonnenen Daten und die Kollektivgruppen-Daten in ein Intervall von 95 % Sicherheit (Fig. 2). Während die Quecksilber-Resistenz ganz allgemein unter den vorliegend verwendeten Bedingungen mit der mer-Expression korreliert ist, zeigte deshalb die Resistenz gegenüber PMA bei der Messung in flüssigen Minimal-Medien die direkteste Beziehung zur mer-Expression.

In scharfem Gegensatz zu diesen Ergebnissen haben Studien in E. coli ergeben, daß mit steigender mer-Gen-Dosis kein Anstieg der Hg$^r$ gezeigt werden kann; die Resistenz wies keine Korrelation mit den Werten für die mer-Expression auf (23, 25). Obwohl man gefunden hat, daß die Quecksilber-Reduktase-Aktivitäten mit der Kopienzahl von mer in E. coli ansteigt, war bei diesen Anstiegen keine gleichzeitige Erhöhung der Hg$^r$ der ganzen Zellen (23, 25) manifest. Weitere Untersuchungen ergaben, daß Hg$^r$ durch den Hg(II)-Transport in die ganzen Zellen limitiert war; der Transport erreichte diesen Ergebnissen zufolge einen sättigungspunkt (31). Weil MerT, eines der Transportproteine, wahrscheinlich ein integrales Protein der inneren Membran ist (15, 21, 25), wurde vorgeschlagen, daß eine Limitation bezüglich der Verfügbarkeit von Insertionsorten für MerT in der Membran für das beobachtete Fehlen der Korrelation zwischen Hg$^r$ und der mer-Expression verantwortlich sein könnte (23, 31). Auch eine limitierte Expression von merPT oder energetische Überlegungen bezüglich des Transports wurden als Erklärungen für die Begrenzung von Hg$^r$ trotz ansteigender mer-Kopienzahl in E. coli angeboten (31).

Die in den oben zitierten studien untersuchte Hg$^r$ von E. coli-Stämmen wurde entweder auf komplexen und/oder verfestigten Medien bestimmt. Sulfhydrylgruppen in komplexen Medien sollten erwartungsgemäß einen gewissen Anteil des verfügbaren Hg(II) binden. Die reale Konzentration an Hg(II), der die Zellen auf Agarplatten ausgesetzt werden, liegt infolge der asymmetrischen physiochemischen Bedingungen von auf einem festen Substrat wachsenden Zellen ebenfalls beinahe mit Sicherheit unterhalb der erwarteten Konzentration. Unterschiede der Hg$^r$ zwischen verschiedenen Stämmen können unter diesen Umständen durchaus abgeschwächt werden, insbesondere, wenn nur leichte Anstiege der Gendosis untersucht werden. Diese Überlegungen könnten auch die Abnahme der Proportionalität erklären, die zwischen der Hg$^r$/PMA$^r$ und der mer-Expression von P. putida::mer-Isolaten auf verfestigten Medien beobachtet wurde (Fig. 2). Auch könnte, da Hg(II) seiner Flüchtigkeit wegen schneller aus flüssigen Medien verloren geht als PMA, eine bessere Korrelation zwischen Resistenz und Expression in PMA-haltigen, flüssigen Medien erzielt werden, was ja vorliegend beobachtet wurde (Fig. 2).

Beim Einsatz von P. putida als genetischem Hintergrund wurde erfindungsgemäß festgestellt, daß die Hyperexpression von mer eine erhöhte Quecksilber-Resistenz verlieh. Ähnliche Versuche in E. coli waren erfolglos, zeigten jedoch die Transportlimitationen in diesem Organismus auf (31). Unterschiede in der Membranzusammensetzung infolge der genetischen Divergenz dieser beiden Organismen und/oder die Zusammensetzung der Medien könnte die Verschiedenheit dieser Ergebnisse erklären. Falls die Membrankapazität für mer-codierte Transportproteine in P. putida signifikant erhöht ist, könnte dies die beobachtete direkte Korrelation der mer-Expression mit der Resistenz zur Folge haben.

### Beispiel 6. Bestimmung der Wachstumsraten in Gegenwart von Quecksilber

Wachstumskurven für die elterlichen, gegenüber Hg empfindlichen (Hg$^s$) Stämme P. putida KT2442 und F1 und deren KT2442::mer-73, F1::mer-1-1 und KT2442-Tn501-132 Hg$^r$-Derivate wurden gemessen, um die Wirkung der merTPAB-Hyperexpression auf die Wachstumsrate der Kultur zu bestimmen.

Hyperexprimierende merTPAB-Stämme zeigten keine signifikante Wirkung der Hg-Exposition auf ihre Wachstumsrate, wenn sie in HgCl$_2$-haltigen LP121-Medien inokuliert wurden, während beide elterlichen P. putida und das Tn501-Derivat unter identischen Bedingungen nicht wachsen konnten (Fig. 3). Da gezeigt worden ist, daß die merTPAB-Expression konstitutiv ist, könnte ein Überleben dieser Stämme auf anfängliche Hg-Exposition hin erwartet werden. Jedoch ist für den Tn501-haltigen Stamm eine Induktion mit Hg erforderlich, damit das durch Tn501 codierte mer-Operon exprimiert werden kann (39); werden die Zellen toxischen Konzentrationen von Hg(II) ausgesetzt, verhindert dies aller Wahrscheinlichkeit nach das Zellwachstum, bevor die mer-Induktion anspringt, wobei gleichzeitig ein Hg$^s$-Phänotyp vorgetäuscht wird (Fig. 3).

Auch die Zugabe von HgCl$_2$ zur midlog-Phase führte zu keiner nennenswerten Wirkung auf die Wachstumsrate von ::mer-73 oder -1-1, während die elterlichen Hg$^s$-Kontrollkulturen einen sofortigen Stillstand des Zellwachstums zeigten und eine induzierte P. putida KT2442:Tn501-132-Kultur eine leicht erhöhte Wachstumsrate aufwies (Fig. 3).

Weiter zurückliegende Wachstumsstudien mit dem induzierbaren mer-Operon vom Wildtyp pDU1358 mit breitem Wirkungsspektrum in E. coli zeigten, daß mit Hg(II)- oder PMA-Konzentrationen unterhalb des toxischen Bereiches behandelte Kulturen mit höheren Raten wachsen als jene, die später toxischen Konzentrationen dieser Verbindungen ausgesetzt wurden (11). P. putida::mer-Stämme` die das alterierte, von pDU1358 stammende mer-System enthalten, zeigten dagegen auch dann, wenn sie mit toxischen Hg-(II)-Konzentrationen behandelt wurden, eine genauso große Wachstumsrate wie unbehandelte Kontrollen (Fig. 3).

### Beispiel 7. Verifizierung einer stabilen merTPAB-Transposition

KT2442::mer-Isolate (-67, -73, -2-4, -2-49, -1-121) und F1::mer-Isolate (-1-1, -1-7, -1-22, -10, -13) von P. putida wurden durch Southern-Hybridisierung (37) analysiert, um die Transposition des mer-Operons zu sichern und die Einmaligkeit der jeweiligen Transpositionsereignisse festzustellen.

Gereinigte pUTHg-DNA wurde mit SfiI verdaut, wodurch ein 3,2 kB-Fragment erzeugt wird, das unter Ausschluß der verkürzten IS50-Sequenzen für die merTPAB-Gene codiert (12). Genomische DNA, die aus den P. putida:mer-Isolaten isoliert worden war, wurde auf die gleiche Weise mit SfiI oder EcoRI geschnitten, für das es im mobilisierten mini-Tn5-mer-TPAB-Konstrukt eine einzelne schnittstelle gibt (12). Genomische DNA der elterlichen Stämme P. putida KT2442 und F1 wurde ebenfalls mit SfiI verdaut. Genomische DNA von P. putida KT2442::Tn501-132 wurde mit AvaI verdaut, wodurch ein 2,7 kB langes Fragment entsteht, das die Tn501 merTPAD-Gene enthält (3, 4, 21).

Die gesamte verdaute DNA wurde einzeln entweder mit dem isolierten 3,2 kB langen pUTHg-Fragment, das für merTPAB codiert, oder mit pGP704 als Sonde zusammengegeben, das die pUTHg-Vektor-Sequenzen (abzüglich mini-Tn5 und tnp*) enthält (12, 20).

Die Ergebnisse zeigten, daß beim Zusammengeben mit der mer-Sequenz in allen SfiI-verdauten P. putida::mer-Proben ein 3,2 kB langes, hybridisierendes Fragment vorhanden war, das der durch SfiI erzeugten pUTHg-mer-DNA entspricht, was zeigt, daß merTPAB tatsächlich durch diese Isolate codiert wird (Fig. 4). Fragmente mit höherem Molekulargewicht aus diesen Verdauungen, die geringere Hybridisierung zeigen, sind wahrscheinlich die Folge von unvollständiger SfiI-Verdauung der hybridisierten DNA (Fig. 4). Die DNA, die KT2442-Tn501 enthielt, zeigte beim Zusammengeben mit merTPAB als Sonde Hybridisierung eines Fragmentes, das der vorhergesagten Größe der Tn501-merTPAD-DNA entspricht (3, 4, 21). Die elterlichen Stämme KT2442 und F1 zeigten erwartungsgemäß keine Hybridisierung mit der mer-Sonde (Fig. 4).

Die Hybridisierung der mer-Sonde mit P. putida::mer-DNA, die mit EcoRI verdaut worden war, führte zu zwei hybridisierenden Fragmenten pro Isolat; alle Fragmente unterschieden sich in ihrer Mobilität (KT2442-Derivate sind in Fig. 4 dargestellt). Demzufolge trat die mer-Transposition in diesen Stämmen nur einmal auf und resultierte in der Integration in einer einzelnen, nur einmal vorkommenden Wirtssequenz.

Beim Hybridisierungsversuch des gleichen DNA-Satzes mit dem mer-Klonierungsvektor pGP704 zeigte nur die pUTHg-Kontrolle positive Hybridisierung (Daten nicht gezeigt). Das Vorliegen von merTPAB in den P. putida::mer-Abkömmlingen ist also nicht die Folge einer Kointegration, da keine Vektorsequenzen beobachtet wurden, sondern zeigt echte Transpositionsereignisse.

### Beispiel 8. Wachstum und Dioxygenase-Aktivität von P. putida F1::mer-Isolaten auf PMA.

Da P. putida F1::mer-Isolate sowohl die durch das Transposon codierte Organoquecksilber-Lyase als auch einen durch ihre Chromosomen spezifizierten Katabolismus für Benzol beinhalten (10), wurden sie daraufhin untersucht, ob sie sowohl Hg(II) aus PMA abspalten als auch die gebildete Benzolgruppe als einzige Kohlenstoff- und Energiequelle nutzen könnten. Deshalb wurde vorliegend die Fähigkeit von 21 P. putida F1::mer-Isolaten analysiert, auf M63-Agar mit 160 ug/ml PMA als einziger Kohlenstoffquelle zu wachsen. Auftretende Kolonien wurden durch einen Farbtest mit 0,5 M Katechol auf das Vorliegen von Aktivität eines der am Benzolabbau beteiligten Enzyme, Katechol-2,3-Dioxygenase (9), getestet.

Beide Kontrollen, P. putida F1 und P. putida KT2442::mer73, konnten nicht auf PMA wachsen (Tabelle 3). P. putida F1 besitzt keine Hg$^r$ mit breitem Wirkungsspektrum und ist deshalb PMA-empfindlich (PMA$^s$)

EP 0 585 652 A2

(Fig. 1). Darüberhinaus könnte PMA deshalb für P. putida F1 nicht als Kohlenstoffquelle verfügbar sein, weil dieser Organismus nicht für eine Organoquecksilber-Lyase codiert und sein Aromaten-Katabolismus PMA nicht als Substrat erkennen sollte. P. putida KT2442::mer-73 dagegen codiert für PMA$^r$ (Fig. 1) und kann deshalb Benzol bilden, kann jedoch nicht auf diesem Substrat wachsen, da ihm ein Benzol-Abbau-Pathway fehlt (Tabelle 3).

Neun der getesteten 21 P. putida F1::mer-Stämme konnten auf M63-PMA-Medien wachsen und zeigten auf ihnen Dioxygenase-Aktivität (Stämme, für die Quecksilber-Reduktase-Bestimmungen vorgenommen wurden, sind in Tabelle 2 dargestellt). Die Wachstumsunterschiede der Stämme auf PMA wiesen keine offensichtliche Korrelation zu den gemessenen Werten für die merA-Expression auf (vergleiche Tabellen 2 und 3). Unterstellt man, daß vergleichbare Reduktase-Werte zwischen ::mer-Stämmen eine Indikation für entsprechende Lyase-Werte sind (da sowohl merA als auch merB in allen diesen Stämmen vom gleichen Promotor exprimiert werden), dann kann die Tatsache, daß sowohl mer-Expressoren mit hohen Werten als auch solche mit niedrigen Werten auf PMA wachsen, nicht durch Bezug auf entsprechenden Werte der Lyase-Aktivität erklärt werden: Die Lyase-Aktivität hätte nicht zu niedrig sein können, um das Wachstum auf dem gebildeten Benzol zu ermöglichen. Gerade diese Frage scheint durch die vorliegenden Daten nicht klärbar zu sein und erfordert weitere Untersuchungen.

Die scheinbare Unvereinbarkeit zwischen dem beobachteten Wachstum auf 160 ug/ml PMA in M63-Minimalmedium und einer maximalen PMA$^r$ von 80 ug/ml auf LP121 läßt sich am ehesten durch einen Zusammenhang mit der jeweiligen Medium-Zusammensetzung erklären. Da LP121 den Zellen phosphatlimitierende Bedingungen (100 uM) bietet, stehen diese wahrscheinlich unter Streß und zeigen deshalb eine niedrigere PMA$^r$ als unter den bezüglich Phosphat unlimitierenden Bedingungen des M63-Mediums. In jedem Fall gelingt es mit Hilfe von Abkömmlingen, die Wachstum auf PMA zeigen, die Aktivitäten von Hg$^r$ mit breitem Wirkungsspektrum mit den endogenen Abbau-Fähigkeiten von P. putida F1 (10) zu verbinden und einen neuen katabolen Pathway zu erzeugen.

Die vorliegende Erfindung stellt damit zum ersten Mal Organismen zur Verfügung, in denen die Überexpression von für die Quecksilberentgiftung benötigten Genen mit einem deutlichen Anstieg der Quecksilber-Resistenz einhergeht.

Zwar ist bereits früher die Überexpression in E. coli insoweit gelungen, als die Kopienzahl des mer-Gens erhöht werden konnte; dies hatte jedoch keinen Anstieg der Quecksilber-Resistenz zur Folge. Es konnte gezeigt werden, daß der Hg(II)-Transport unter diesen Bedingungen in E. coli limitierend war. Eines der Transport-Proteine ist in die Zellmembran integral insertiert, und wahrscheinlich ist die Zahl der für Hg-(II)-Transportproteine verfügbaren Insertionsorte begrenzt. Deshalb können mer-überexprimierende E. coli-Stämme Hg(II) unter hohen Konzentrationen nicht schnell genug eintransportieren (und dann reduzieren), um sich selbst vor der Toxizität gegenüber dem Kation zu schützen.

Erfindungsgemäß wurde nun mit P. putida ein Organismus bereitgestellt, in dem die Überexpression und die Resistenz gekoppelt sind. Der Grund für die Kopplung könnte darin liegen, daß die Membranen von P. putida mehr Insertionsstellen für die mer-Transportproteine bereitstellen als die von E. coli, sodaß der Eintransport in die Zelle und damit die Hg(II)-Reduktionsrate gesteigert werden.

Ein zweiter wichtiger Vorteil der erfindungsgemäßen Systeme ist die konstitutive Expression der Proteine. Die Zellen sterben deshalb nicht ab, bevor die Hg(II)-Reduktion induziert ist, wie es die Wildtyp-Isolate tun. Auch in einer Umgebung, die mit niedrigen Konzentrationen an Hg(II) kontaminiert ist, sind Organismen mit konstitutiver Expression überlegen: Während derartige Konzentrationen nicht ausreichen, um in den Wildtypen die Induktion der Proteinsynthese und Quecksilber-Reduktion zu bewirken, reduzieren die erfindungsgemäßen Stämme auch Hg(II) in hohen Verdünnungen. Dies ist von großem Vorteil, da sich gezeigt hat, daß Hg, auch wenn es in hochverdünnten und damit (noch) ungefährlichen Konzentrationen in der Umgebung vorhanden ist, in höheren Organismen akkumuliert und damit langfristig giftig und belastend ist.

Lebendmaterial

P. putida KT2442; Gene, 16 (1981) 237-247
P. putida F1; J. Biol. Chem., 264 (1989) 14940-14946
E. coli SM10 lambda-pir (pUT-Hg); vgl. J. Bacteriol., 172 (1990) 6568-6572

**LITERATUR**

**1. Bauchop, T., and S. R. Eldsen.** 1960. The growth of microorganisms in relation to their energy supply. J. Gen. Microbiol. **23**:457-469.

**2. Begley, T. P., A. E. Walts, and C. T. Walsh.** 1986. Bacterial organomercurial lyase:overproduction, isolation and characterization. Biochem. **25:**7186-7192.

**3. Brown, N. L., S. J. Ford, R. D. Pridmore, and D. C. Fritzinger.** 1983. Nucleotide sequence of a gene from the *Pseudomonas* transposon Tn*501* encoding mercuric reductase. Biochem. **22:**4089-4095.

**4. Brown, N. L., T. K. Misra, J .N. Winnie, A. Schmidt, M. Seiff, and S. Silver.** 1986. The nucleotide sequence of the mercuric resistance operons of plasmid R100 and transposon Tn*501*: further evidence for *mer* genes which enhance the activity of the mercuric ion detoxification system. Mol. Gen. Genet. **202:**143-151.

**5. Boyer, H. W., and D. Roullard-Dussoix.** 1969. A complementation analysis of the restriction and modification of DNA in *Escherichia coli*. J. Molec. Biol. **41:**459-472.

**6. Figurski, D., and D. R. Helinski.** 1979. Replication of an origin-containing derivative of plasmid RK2 dependent on a piasmid function provided in *trans*. Proc. Natl. Acad. Sci. USA **76:**1648-1652.

**7. Foster, T. J., and F. Ginnity.** 1985. Some mercurial resistance plasmids from different incompatibility groups specify *merR* regulatory functions that both repress and induce the *mer* operon of plasmmid R100. J. Bacteriol. **162:**773-776.

**8. Fox, B., and C. T. Walsh.** 1982. Mercuric reductase. Purification and characterization of a transposon-encoded flavoprotein containing an oxidation-reduction active disulfide. J. Biol. Chem. **257:**2498-2503.

**9. Franklin, F. C. H., M. Bagdasarian, M. M. Bagdasarian, and K. N. Timmis.** 1981. Molecular and functional analysis of the TOL plasmid pWWO from *Pseudomonas putida* and cloning of genes for the entire regulated aromatic ring *meta* cleavage pathway. Proc. Natl Acad. Sci. **78:**7458-7462.

9a. Frischmuth, A., 1991. Entwicklung eines Bioreaktors zur Entfernung von Quecksilber aus wäßrigen Medien durch einen aktiven mikrobiellen Prozeß. Dissertation TU Braunschweig.

**10. Gibson, D. T., J.R. Koch, and R. E. Kallio.** 1968. Oxidative degradation of aromatic hydrocarbons by microorganisms. I.enzymatic formation of catechol from benzene. Biochemistry **7:**2653-2662.

**11. Griffin, H. G., T. J. Foster, S. Silver, and T. K. Misra.** 1987. Cloning and DNA sequence of the mercuric- and organomercurial-resistance determinants of plasmid pDU1358. Proc. Natl. Acad. Sci. USA **84:**3112-3116.

**12. Herrero, M., V. da Lorenzo, and K. N. Timmis.** 1990. Transposon vectors containing non-antibiotic resistance selection markers for cloning and stable chromosomal insertion of foreign genes in gram-negative bacteria. J. Bacteriol. **172:**6557-6567.

**13. Itoh, Y., and D. Haas.** 1985. Cloning vectors derived from the *Pseudomonas* plasmid pVS1. Gene **36:**27-36.

**14. Kreuzer, K., C. Pratt, and A. Torriani.** 1975. Genetic analysis of regulatory mutants of alkaline phosphatase. Genetics **81:**459-468.

**15. Lund, P. A., and N. L. Brown.** 1987. Roie of the *merT* and *merP* gene products of transposion Tn*501* in the induction and expression of resistance to mercuric ions. Gene **52:**207-214.

**16. Lund, P. A., and N. L. Brown.** 1989. Regulation of transcription in *Escherichia coli* from the *mer* and *merR* promoters in the transposon Tn*501*. J. Mol. Biol. **205:**343-353.

**17. Mason, R. P., and W. F. Fitzgerald.** 1990. Alkylmercury species in the equatorial Pacific. Nature **347:**457-459.

**18. Mermod, N., P. R. Lehrbach, R. H. Don, and K. N. Timmis.** 1986. Gene cloning and mainipulation in *Pseudomonas*, p. 325-355. *In* J.R. Sokatch (ed.), The bacteria, vol. 10. Academic Press, Inc., New York.

**19. Miller, J. H.** 1972. Experiments in molecular genetics. Cold Spring Harbor Laboratory, Cold Spring Harbor, N.Y.

**20. Miller, V. L., and J. J. Mekalanos.** 1988. A novel suicide vector and its use in the construction of insertion mutations:osmoregulation of outer membrane proteins and virulence determinants in *Vibrio cholerae* requires *toxR*. J. Bacteriol. **170:**2575-2583.

**21. Misra, T. K., N.L. Brown, D. Fritzinger, R.D. Pridsmore, W.M. Barnes, L. Haberstroh, and S. Silver.** 1985. Mercuric ion resistance operons of plasmid R100 and transposon Tn*501*: the beginning of the operon including the regulatory region and the first two structural genes. Proc. Natl. Acad. Sci. USA **81:**5975-5979.

**22. Mukhopadhyay, D., H. Yu, G. Nucifora, and T. K. Misra.** 1991. Purification and functional characterization of MerD. J. Biol. Chem. **266:**18538-18542.

**23. Nakahara, H., T. G. Kinscherf, S. Silver, T. Miki, A. M. Easton and R. H. Rownd.** 1979. Gene copy number effects in the *mer* operon plasmid NR1. J. Bacteriol. **138:**284-287.

**24. Nakahara, H., S. Silver, T. Miki, and R. H. Rownd.** 1979. Hypersensitivity to Hg(II) and hyperbinding activity associated with cloned fragments of the mercurial resistance operon of plasmid NR1. J.

Bacteriol. **140**:161-166.

**25. NiBhriain, N., S. Silver, and T. J. Foster.** 1983. Tn*5* insertion mutants in the mercuric ion resistance genes derived from plasmid R100. J. Bacteriol. **155**:690-703.

**26. Niebor, E., and D. H. S. Richardson.** 1980. The replacement of the nondescript term "heavy metals" by a biologically and chemically significant classification of metal ions. Environ. Pollut. Ser. B **1**:3-26.

**27. Nriagu, J. O., and J. M. Pacyna.** 1988. Quantitative assessment of worldwide contamination of air, water and soils by trace metals. Nature **333**:134-139.

**28. Nucifora, G., L. Chu, S. Silver, and T. K. Misra.** 1989. Mercury operon regulation by the *merR* gene of the organomercurial resistance system of plasmid pDU1358. J. Bacteriol. **171**:4241-4247.

**29. Nucifora, G., S. Silver, and T. K. Misra.** 1989. Down regulation of the mercury resistance operon by the most promoter-distal gene *merD*. Mol. Gen. Genet. **220**:69-72.

**30. OHalloran, T., and C. Walsh.** 1987. Metalloregulatory DNA-binding protein encoded by the *merR*gene: isolation and characterization. Science **235**:211-214.

**31. Philippidis, G. P., L-H. Malmberg, W-S. Hu, and J. L. Schottel.** 1991. Effect of gene amplication on the mercuric reduction activity of *Escherichia coli*. Appl. Environ. Microbiol. **57**:3558-3564.

**32. Ramos, J.L., Wasserfallen, A., Rose, K., and Timmis, K.N.** 1987. Redesigning metabolic routes: manipulation of the TOL plasmid pathway for the catabolism of alkylbenzoates. Science **235**:593-596.

**33. Richards, E** 1987. Preperation of genomic DNA from bacteria. Miniprep of bacterial genomic DNA, p. 1-2. unit 2.4.1. *In* F.M. Ausubel, R. Brent, R.E. Kingston, D.D. Moore, J.G. Seidman, J.A. Smith, and K. Struhl (ed.), Current protocols in molecular biology. John Wiley & Sons, Inc. New York.

**34. Rojo, F., Pieper, D.H., Engesser, K.H., Knackmuss, H.J., and Timmis, K.N.** 1987. Assemblage of an *ortho* cleavage route for the simultaneous degradation of chloro- and methylaromatics. Science **235**:1395-1398.

**35. Sambrook, J., E. F. Fritsch, and T. Maniatis.** 1989. Molecular cloning: a laboratory manual, 2nd ed. Cold Spring Harbor Laboratory Press, Cold Spring Harbor, N.Y.

**36. Schottel, J. L.** 1978. The mercuric and organomercuric detoxifying enzymes from a plasmid-bearing strain o*f Escherichia coli*. J. Biol. Chem. **253**:4341-4349.

**37. Southern, E. M.** 1975. Detection of specific DNA sequences among DNA fragments seperated by gel electrophoresis. J. Mol. Biol. **98**:503-517.

**38. Stanisich, V. A., P. M. Bennett, and M. H. Richmond.** 1977. Characterization of a translocation unit encoding resistance to mercuric ions that occurs on a nonconjugative plasmid in *Pseudomonas aeruginosa*. J. Bacteriol. **129**:1227-1233.

**39. Summers, A. O.** 1986. Organization, expression, and evolution of genes for mercury resistance. Ann. Rev. Microbiol. **40**:607-634.

**40. Summers, A. O., and L. I Sugarman.** 1974. Cell-free mercury(II)reducing activity in a plasmid-bearing strain of *Escherichia coli*. J. Bacteriol. **119**:242-249.

**41. Tezuka, T., and K. Tonomura.** 1978. Purification and properties of a second enzyme catalyzing the splitting of carbon-mercury linkages from mercury-resistant *Pseudomonas* K-62. J. Bacteriol. **135**:138-143.

**42. Timmis, K.N.** 1990. Genetic construction of novel metabolic pathways: degradation of xenobiotica, p. 69-83. *In* K. Mosbach (ed.), Colloquium 41: Molecular basis of bacterial metabolism. Springer-Verlag, Berlin.

**43. Young, R. A., and R. W. Davis.** 1983. Efficient isolation of genes by using antibody probes. Proc. Natl. Acad. Sci. **80:** 1194.

**Patentansprüche**

**1.** Bakterienstamm, dadurch gekennzeichnet, daß er

(i) in der Lage ist, für die Quecksilber-Reduktion notwendige Gene (mer-Gene) konstitutiv zu überexprimieren,
(ii) resistent gegen Hg(II)-Verbindungen ist.

**2.** Bakterienstamm nach Anspruch 1, dadurch gekennzeichnet, daß er zusätzlich

(iii) Toluol und Benzol sowie Toluol- und Benzolgruppen organischer Substanzen abbauen kann.

**3.** Bakterienstamm nach Anspruch 1 oder 2, dadurch gekennzeichnet, daß es sich um einen P. putida-Stamm handelt.

4. Bakterienstamm nach Anspruch 3, dadurch gekennzeichnet, daß es sich um P. putida F1 oder um P. putida. KT2442 handelt.

5. Bakterienstamm nach einem der Ansprüche 1 bis 4, dadurch gekennzeichnet, daß er die mer-Gene in seiner genomischen DNA insertiert enthält.

6. Bakterienstamm nach Anspruch 5, dadurch gekennzeichnet, daß in den mer-Genen die Regulatorgene oder -sequenzen ganz oder teilweise deletiert sind.

7. Bakterienstamm nach Anspruch 5 oder 6, dadurch gekennzeichnet, daß die mer-Gene durch Transposition von pUTHg in die Wirts-DNA insertiert worden sind.

8. P. putida KT 2442 x pUTHg oder P. putida F1 x. pUTHg,

9. P. putida KT 2442 :: mer-73, hinterlegt unter der DSM Nr.7209 oder P. putida F1 :: mer-1-1, hinterlegt unter der DSM Nr.7208

10. Verfahren zum Herstellen eines Bakterienstammes nach einem der Ansprüche 1 bis 9 durch ungezielte Transposon-Mutagenese mit einem mer-Gene enthaltenden Transposon.

11. Verfahren nach Anspruch 10, dadurch gekennzeichnet, daß das Transposon
      (i) eine kurze Endsequenz aufweist,
      (ii) nur einmal in den Akzeptor bzw. Rezipienten transponiert wird und dort stabil integriert verbleibt,
      (iii) keine mer-Regulatorgene besitzt.

12. Verfahren nach Anspruch 10 oder 11, dadurch gekennzeichnet, daß ein transposonenhaltiges Plasmid aus einem E. coli-Donor, z.B. SM10 λ-pir, durch Mobilisierung mit einer biparentalen Filter-Mating-Technik in den entsprechenden Akzeptor transferiert wird.

13. Verfahren nach einem der Ansprüche 10 bis 12, dadurch gekennzeichnet, daß die Konjugationsprodukte auf Quecksilber-Resistenz selektioniert werden.

14. Verfahren nach Anspruch 13, dadurch gekennzeichnet, daß das Selektionieren durch Screenen der Isolate mit einem Medium erfolgt, das Phenylquecksilberacetat enthält.

15. Verfahren nach Anspruch 14, dadurch gekennzeichnet, daß das Screenen der Isolate mit steigenden Konzentrationen Phenylquecksilberacetat im Medium erfolgt.

Fig. 1

Maximale HgCl$_2$- oder PMA-Resistenz (µg/ml)

Fig. 2

Fig. 3

Fig. 4

A.

Kb    1 23456   7  8 9 10

23.1-
9.4-
6.6-
4.4-
2.3-
2.0-

B.

Kb    1 2345678   9

23.1-
9.4-
6.6-
4.4-
2.3-